# EUROPEAN PATENT APPLICATION

(11) **EP 1 994 929 A1**
(43) Date of publication of application: **26.11.2008**
(21) Application number: 07730324.6
(22) Date of filing: 14.02.2007
(51) Int. Cl.: A61K 31/185, A61K 31/47, A61P 27/02

(54) **USE OF DERIVATIVES OF NAPHTHALENESULPHONIC OR QUINOLINESULPHONIC ACID IN THE TREATMENT OF VASOPROLIFERATIVE OCULAR DISEASES**

(30) Priority: 14.02.2006 ES 200600330
(71) Applicant: ITALFARMACO, S.A., E-28108 Alcobendas (ES)
(72) Inventor: MOSCOSO DEL PRADO, Jaime, E-28108 Alcobendas - Madrid (ES)
(74) Representative: de Justo Vàzquez, Jorge M.
(86) International application number: PCT/ES2007/000083
(87) International publication number: WO 2007/093656

(57) **Abstract**

The invention relates to the use of derivatives of sulphonic acid (I) or one of the pharmaceutically- acceptable prodrugs or salts thereof in the preparation of a medicament for the treatment of vasoproliferative ocular diseases, said sulphonic acid having formula (I).

## Description

### FIELD OF THE INVENTION

This invention relates to the use of derivatives of naphthalenesulfonic or quinolinesulfonic acid in the treatment of vasoproliferative ocular diseases.

### BACKGROUND OF THE INVENTION

Neovascularization within the eye, primarily in the retina or in the choroidea, contributes to the loss of vision in various ocular diseases. These vasoproliferative pathologies affect people in various stages of life, from birth to old age, and are the cause of many cases of blindness.

Retinal vasoproliferative diseases are the most common cause of severe loss of vision in the copulation under 60 years of age in developed countries. The patients with the highest risk are diabetics, premature children or patients with occlusion of the retinal vein. Diabetic retinopathy can take two forms: non-proliferative retinopathy, in which small capillaries of the retina rupture and begin to lose blood, forming small sacks in which the blood proteins are deposited in the area surrounding each rupture; and proliferative retinopathy, in which the damage in the retina stimulates the formation of abnormal blood vessels, hence causing scarring and, on occasions, detachment of the retina.

The macular degeneration associated with ageing is one of the primary causes of loss of vision in people older than 65. Two forms are observed: atrophic (dry) macular degeneration, in which pigment alterations are observed in the macular region without raised scars or the presence of hemorrhages or exudates; and exudative (wet) macular degeneration, in which a choroideal subretinal network of neovascularization is formed, and the lesion finally contracts, leaving a raised and well-defined scar in the posterior pole.

Ocular neovascularization is fundamentally produced by hypoxic conditions which, in turn, trigger the synthesis and release of various growth factor, the activity of many of which promotes angiogenesis. Among these factors are fibroblast growth factor (FGF), epidermal growth factor (EGF), insulin-like growth factor (IGF), and vascular endothelial growth factor (VEGF). Of these factors, VEGF is considered to be the primary culprit in retinal neovascularization, and various studies have been conducted with the aim of demonstrating that the inhibition of VEGF blocks neovascularization in the retina (Ozaki H et al. Intravitreal sustained release of VEGF causes retinal neovascularization in rabbits and breakdown of the bloodretinal harrier in rabbits and primates. Exp Eye Res 1997, 64:505-17; Kim KJ et al. Inhibition of vascular endothelial growth factor-induced angiogenesis suppresses tumour growth in vivo. Nature 1993, 362:841-4; Ozaki H et al. Blockade of vascular endothelial cell growth factor receptor signaling is sufficient to completely prevent retinal neovascularization. Am J Pathol 2000, 156:697-707; Aiello LP et al. Suppression of retinal neovascularization in vivo by inhibition of vascular endothelial growth factor (VEGF) using soluble VEGF-receptor chimeric proteins. Proc Natl Acad Sci USA 1995, 92:10457-61; Robinson GS et al. Oligodeoxynucleotides inhibit retinal neovascularization in a murine model of proliferative retinopathy. Proc Natl Acad Sci USA 1996, 93:4851-6; Sone H et al. Effects of intraocular or systemic administration of neutralizing antibody against vascular endothelial growth factor on the murine experimental model of retinopathy. Life Sci 1999, 65:2573-80; Boden KT et al. Therapy of angioproliferative retinopathy with soluble VEGF- and Tie-2-receptors in a mouse model. Ophthalmologe 2001, 98:S23).

Other unspecific inhibitors have also been tested with the idea of blocking different mechanisms simultaneously. For example, oxoindolinone MAE 87, a compound inhibitor of certain VEGF, IGF, FGF and EGF receptors, has been tested (Unsoeld, A.S. et al. Local injection of receptor tyrosine kinase inhibitor MAE 87 reduces retinal neovascularization in mice. Molecular Vision 2004; 10:468-75).

On the other hand, the role of FGF has not been considered to be as relevant, and there are no references to the inhibition thereof as a method for blocking neovascularization.

According to WO 2004/078704, certain derivatives of naphthalenesulfonic or quinolinesulfonic acid of formula (I) which comprise an optionally substituted polar sulfonic/sulfonate group and are situated in certain positions in the aromatic ring inhibit the activity of the FGFs. There are studies on their inhibitory properties with respect to the mitogenic activity induced by aFGF on fibroblasts in culture, to angiogenesis in animals and to the formation of tumors in animals as well. It is concluded that said compounds could potentially be useful in the treatment of cancer, especially in the treatment of solid tumors, and in the treatment of non-tumoral, angiodependent cutaneous diseases, etc.; rheumatoid arthritis; endometriosis; obesity; arteriosclerosis, restenosis; etc. However, there is no mention of the idea of their being useful in the treatment of vasoproliferative ocular diseases, nor is proof provided thereof.

### SUMMARY OF THE INVENTION

Until a few years ago, the only approved treatment for the vasoproliferative ocular diseases consisted in the destruction of the peripheral retina by laser or cryocoagulation with the purpose of diminishing the hypoxic tissue and hence reducing the neovascularization.

The success of these treatments tends to be limited, achieving at best the halting of the progression of the disease or an improvement of vision for a limited time and in a smaller percentage. There is therefore the need for nondestructive and/or more effective treatments for these vasoproliferative diseases.

The inventors of the present invention found that, surprisingly, the derivatives of sulfonic acid of formula (I), described in WO 2004/078704 only as FGF inhibitors, permit an inhibition of the neovascularization in the eye to a degree which is comparable to that obtained with VEGF inhibitors or unspecific inhibitors and, as a result, are useful in the treatment of vasoproliferative ocular diseases.

As a consequence, a first aspect of the present invention relates to the use of derivatives of the sulfonic acid of formula (I): 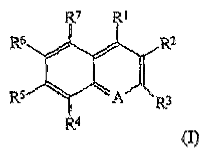 where
A is N or a group of formula CR⁸;
R¹, R², R³, R⁴ R⁵, R⁶, R⁷ and R⁸, independently of each other, represent H, SO₃R⁹, OR¹⁰, CO₂R¹⁰, NR¹⁰R¹¹, NH-CO-R¹² or alkyl C₁-C₆;
R⁹ is H, ammonium or a cation of an alkaline or earth alkaline metal;
R¹⁰ and R¹¹, independently of each other, represent H, alkyl C₁₋C₆ or aryl C₆-C₁₀;
R¹² is OH, alkyl C₁-C₆ or aryl C₆-C₁₀;
with the condition that (a) at least one of R¹ to R⁸ is SO₃R⁹, and (b) at least one of the remaining R¹ to R⁸ is an OR¹⁰, CO₂R¹⁰, NR¹⁰R¹¹ or NH-CO-R¹² group, with R⁹, R¹⁰, R¹¹ and R¹² having the previously-mentioned meanings,
or one of the pharmaceutically acceptable salts or prodrugs thereof, in the preparation of a medication for the treatment of vasoproliferative ocular diseases.

The term "alkyl C₁-C₆" as used in this specification refers to a radical derived from a saturated, linear or branched hydrocarbon having 1 to 6 carbon atoms, for example methyl, ethyl, isopropyl, etc.

The term "aryl C₆-C₁₀" as used in this specification refers to a radical derived from an aromatic hydrocarbon having 6 to 10 carbon atoms, for example phenyl, naphthyl, etc.

The pharmaceutically acceptable salts of the compounds of formula (I) are within the scope of this invention. The term "pharmaceutically acceptable salts" includes the metallic salts or the addition salts which can be used in pharmaceutical forms.

### BRIEF DESCRIPTION OF THE FIGURES

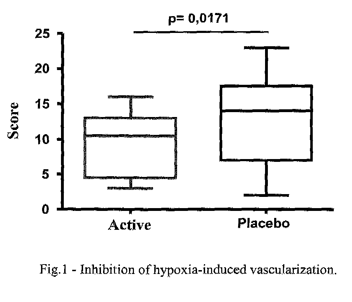

Figure 1 is a graphic showing the score obtained in the study of Example 1 by sodium 5-amino-2-naphthalene sulfonate compared to placebo and illustrates the inhibitory effect of the compound on neovascularization induced by hypoxia of the retina.
Figure 2 presents photographs of histological sections of an eye treated with sodium 5-amino-2-naphthalene sulfonate and its contralateral with placebo, illustrative of the antiangiogenic effect of the compound.

### DETAILED DESCRIPTION OF THE INVENTION

The compounds of formula (I) include derivatives of naphthalenesulfonic acid [compounds of formula (I) wherein A is CR⁸] and derivatives of quinolinesulfonic acid [compounds of formula (I) wherein A is nitrogen].

In one particular embodiment, the compound of formula (I) is a derivative of naphthalenesulfonic acid belonging to the 2-NMS series (derivatives of 2-naphthalenesulfonic acid) wherein:
A is CR⁸;
R¹ is H or alkyl C₁-C₆;
R² is SO₃R⁹;
R³ is H, OH or alkyl C₁-C₆;
R⁴, R⁵, R⁶, R⁷ and R⁸, independently of each other, represent H, OR¹⁰, CO₂R¹⁰, NR¹⁰R¹¹, NH-CO-R¹² or alkyl C₁-C₆;
R⁹ is H, ammonium or a cation of an alkaline or earth alkaline metal;
R¹⁰ and R¹¹, independently of each other, represent H, alkyl C₁-C₆ or aryl C₆-C₁₀;
R¹² is OH, alkyl C₁-C₆ or aryl C₆-C₁₀;
   with the condition that at least one of R⁴, R⁵, R⁶ R⁷
   or R⁸ is OR¹⁰, CO₂R¹⁰, NR¹⁰R¹¹ or NH-CO-R¹², with R¹⁰, R¹¹ and R¹² having the previously-mentioned meanings.

Preferred compounds of the 2-NMS series include compounds of formula (I) wherein:
A is CR⁸, with R⁸ being H or OH;
R¹ is H;
R² is SO₃R⁹, with R⁹ being H or sodium;
R³ is H;
R⁴, R⁵, R⁶ and R⁷, independently of each other, represent H, OH, OCH₃, COOH, NH₂, NHCOCH₃, NHCOC₆H₅, NHCH₃ or N(CH₃)₂;
   with the condition that at least one of R⁴, R⁵, R⁶ or R⁷ is OH, OCH₃, COOH, NH₂, NHCOCH₃, NHCOC₆H₅, NHCH₃ or N(CH₃)₂.

Illustrative examples of compounds of the 2-NMS series include: sodium 5-amino-2-naphthalene sulfonate, sodium 5-acetyl amino-2 naphthalene sulfonate, sodium 5-benzoyl-amino-2-naphthalene sulfonate, sodium 8-amino-2-naphthalene sulfonate, sodium 8-acetyl amino-2-naphthalene sulfonate and sodium 4-hidroxy-6-amino-2-naphthalene sulfonate.

Within this 2-NMS series, the compounds sodium 5-amino-2-naphthalene sulfonate, sodium 5-acetyl amino-2 naphthalene sulfonate, sodium 8-amino-2-naphthalene sulfonate and sodium 4-hidroxy-6-amino-2-naphthalene sulfonate are especially preferred. In particular, sodium 5-amino-2-naphthalene sulfonate is very especially preferred.

In another particular embodiment, the compound of the formula (I) is a derivative of the naphthalenesulfonic acid belonging to the 1-NMS series (derivatives of 1-naphthalenesulfonic acid) wherein:
A is CR⁸;
R¹ is SO³R⁹;
R² is H or alkyl C₁-C₆;
R³ is H, OH or alkyl C₁-C₆;
R⁴, R⁵, R⁶, R⁷ and R⁸, independently of each other, represent H, OR¹⁰, CO₂R¹⁰, NR¹⁰R¹¹, NH-CO-R¹² or alkyl C₁-C₆;
R⁹ is H, ammonium or a cation of an alkaline or earth alkaline metal; R¹⁰ and R¹¹, independently of each other, represent H, alkyl C₁-C₆ or aryl C₆-C₁₀;
R¹² is OH, alkyl C₁-C₆ or aryl C₆-C₁₀;
   with the condition that at least one of R⁴, R⁵, R⁶, R⁷ or R⁸ is OR¹⁰, CO₂R¹⁰, NR¹⁰R¹¹ or NH-CO-R¹², with R¹⁰, R¹¹ and R¹² having the previously-mentioned meanings.

Preferred compounds of the 1-NMS series include compounds of formula (I) wherein:
A is CR⁸;
R¹ is SO₃R⁹, with R⁹ being H or sodium;
R² is H;
R³ is H or OH;
R⁴, R⁵, R⁶, R⁷ and R⁸, independently of each other, represent H, OH, OCH₃, COOH, NH₂, NHCOCH₃, NHCOC₆H₅, NHCH₃ or N(CH₃)₂;
   with the condition that at least one of R⁴, R⁵, R⁶, R⁷ or R⁸ is OH, OCH₃, COOH, NH₂, NHCOCH₃, NHCOC₆H₅, NHCH₃ or N(CH₃)₂.

Illustrative examples of compounds of the 1-NMS series include: sodium 4-amino-1-naphthalene sulfonate, sodium 4-acetyl amino-1-naphthalene sulfonate, sodium 4-benzoyl-amino-1-naphtalene sulfonate, sodium 5-dimethyl amino-1-naphtalene sulfonate and sodium 4-amino-3-hydroxy-1-naphthalene sulfonate.

Within this 1-NMS series, the compound sodium 4-amino-3-hydroxy-1-naphthalene sulfonate is especially preferred.

In another particular embodiment, the compound of formula (I) is a derivative of quinolinesulfonic acid wherein:
A is N;
R¹, R², R³, R⁴, R⁵, R⁶, R⁷ and R⁸, independently of each other, represent H, SO₃R⁹, OR¹⁰, CO₂R¹⁰, NR¹⁰R¹¹, NH-CO-R¹² or alkyl C₁-C₆;
R⁹ is H, ammonium or a cation of an alkaline or earth alkaline metal;
R¹⁰ and R¹¹, independently of each other, represent H, alkyl C₁-C₆ or aryl C₆-C₁₀;
R¹² is OH, alkyl C₁-C₆ or aryl C₆-C₁₀;
   with the condition that (a) at least one of R¹ to R⁶ is SO₃R⁹, and (b) at least one of the remaining R¹ to P⁸ is an OR¹⁰, CO₂R¹⁰, NR¹⁰R¹¹ or NH-CO-R¹² group, with R⁹, R¹⁰, R¹¹ and R¹² having the previously-mentioned meanings.

The compounds of formula (I) or one of its pharmaceutically acceptable salts or prodrugs are known products which are commercially available or can be obtained by means of conventional methods.

The nature of the salt is not critical as long as it is pharmaceutically acceptable. The salts of the compound of formula (I) can be obtained on the basis of organic or inorganic acids or bases by conventional methods well known by those skilled in the art by reacting the appropriate acid or base with the compound of formula (I).

The prodrugs can be obtained, for example, starting from a free hydroxyl group which is converted into an ester or from an amino group which is converted into an amide, using conventional methods well known by those skilled in the art, by reacting a compound of formula (I) with a carboxylic acid, an anhydride or an acyl halide in the presence of a base or catalyst.

As has been stated, the use of the compounds of formula (I) in the preparation of a medication for the treatment of vasoproliferative ocular diseases constitutes the primary aspect of this invention.

The medication can be administered to a mammal, preferably a human being, by means of any formulation which permits contact between the compound of formula (I) and its site of action. This formulation will comprise a therapeutically effective quantity of at least one compound of formula (I) or one of its pharmaceutically acceptable salts or prodrugs, together with at least one pharmaceutically acceptable excipient. Preferably, the formulation of the present invention will comprise at least one compound of formula (I) selected from among sodium 5-amino-2-naphthalene sulfonate, sodium 5-acetyl amino-2-naphthalene sulfonate, sodium 5-benzoyl amino-2-naphthalene sulfonate, sodium 8-amino-2-naphthalene sulfonate, sodium 8-acetyl amino-2-naphthalene sulfonate, sodium 4-hydroxy-6-amino-2-naphthalene sulfonate, sodium 4-amino-1-naphthalene sulfonate, sodium 4-acetyl amino-1-naphthalene sulfonate, sodium 4-benzoyl-amino-1-naphthalene sulfonate, sodium 5-dimethyl amino-1-naphthalene sulfonate and sodium 4-amino-3-hydroxy-1-naphthalene sulfonate and mixtures thereof. More preferably, the formulation will comprise at least one compound selected from among sodium 5-amino-2-naphthalene sulfonate, sodium 5-acetyl amino-2-naphthalene sulfonate, sodium 8-amino-2-naphthalene sulfonate, sodium 4-hydroxy-6-amino-2-naphthalene sulfonate and sodium 4-amino-3-hydroxy-1-naphthalene sulfonate.

The quantity of compound of formula (I) which must be administered in order to treat the vasoproliferative ocular disease in a safe and effective manner will depend on numerous factors, including age and condition of the patient, the severity of the disease, the mode and frequency of administration of the formulation, the compound of formula (I) used, etc.

The formulation which contains the compound of formula (I) can be presented in any pharmaceutical form that is considered suitable -solid, semisolid or liquid, for example- and can be administered by any appropriate mode - orally, parenterally, topically or ocularly, for example. The pharmaceutically acceptable excipients can be selected from among any of those known by an expert in the art and will depend on the pharmaceutical form to be prepared.

The ocular diseases to be treated with the compounds of formula (I) include all of those in which there is neovascularization within the eye, for example proliferative diabetic retinopathy and wet senile macular degeneration.

The following example illustrates the invention and must not be considered as limiting the scope of same.

### EXAMPLE 1

### Inhibition of hypoxia-induced vascularization

A total of thirty C57BL/6J mice were used in a model of retinopathy of prematurity. Seven days after birth, the mice (young) were transferred together with their mothers to a high-oxygen atmosphere (75%) obtained using a mixture of oxygen and air. After 5 days, the animals were transferred to a normal atmosphere. The relative hypoxia produced leads to the release of growth factors which, in turn, cause hypervascularization of the retina. 14-18 hours thereafter, an intravitreous injection of 2 µl of a sterile saline solution of sodium 5-amino-2-naphthalene sulfonate (25 mg/ml) into an eye was performed, and another injection of an equivalent volume of saline solution was performed in the contralateral eye.

Five days after the return to normal atmosphere, the mice were put down by means of an intracardiac injection of dextrane marked with fluorescein (50 mg/ml), the nuclei were removed from both eyes and were fixed for 2 to 6 hours in formaldehyde (4%) at room temperature.

Subsequently, the retina was cut and mounted for observation by fluorescent microscopy, with the vasculature of the retina being assessed. Given that the retina occupies a circular area, in order to perform the assessment, 12 equal radical segments are considered. For the assessment of the central vascularization, the retina is divided into three concentric circles defining three zones: the interior zone around the optic disc, the middle and the exterior zone.

The retinopathy is quantified using a system of multiple assessment in which various morphological parameters associated with retinopathy are assessed:
- Presence of a "crest" in each radial segment. One point for each radial segment with the presence of crests. Scoring from 0 to 12.
- Presumed extraretinal vascularization, indicated by the presence of groupings of "crest-like" structures. One point for each radial segment with groupings. Scoring from 0 to 12.
- Size of the central avascular zone. Scoring from 0 to 3, with 0 meaning an occupation of less than 50% of the interior zone; 1 an occupation of less than 25% of the sum of the interior and middle zone; 2 an occupation of between 25-50% of said sum and 3 an occupation of greater than 50% of same.
- Tortuosity of the vessels, measured as a percentage of tortuous vessels with respect to the total. The scoring ranges from 0 to 3, with 0 meaning no tortuosity, 1 if less than 1/3 of the total are tortuous, 2 if between 1/3 and 2/3 are tortuous, and 3 if more than 2/3 are tortuous.

For each eye, a global assessment was obtained consisting of the sum of the points obtained for each parameter.

The statistical analysis of the data was performed subsequently using a Wilcoxon's test.

The results obtained are as follows:

| | **ACTIVE** | **PLACEBO** |
|---|---|---|
| **N** | 30 | 30 |
| **Median** | 10.5 | 14.0 |
| **Maximum** | 16 | 23 |
| **Minimum** | 3 | 2 |

The difference between the two treatments is highly significant (p = 0.017).

This test demonstrates the ability of the derivatives of sulfonic acid of formula (I) to inhibit hypoxia-induced angiogenesis and, consequently, ocular vascular proliferation.

## Claims

1. Use of the derivatives of sulfonic acid of formula (I) 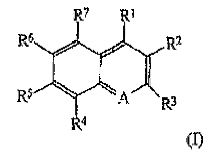 where
A is N or a group of formula CR⁸;
R¹, R², R³, R⁴, R⁵, R⁶, R⁷ and R⁸, independently of each other, represent H, SO₃R⁹, OR¹⁰, CO₂R¹⁰, NR¹⁰R¹¹, NH-CO-R¹² or alkyl C₁-C₆;
R⁹ is H, ammonium or a cation of an alkaline or earth alkaline metal;
R¹⁰ and R¹¹, independently of each other, represent H, alkyl C₁-C₆ or aryl C₆-C₁₀;
R¹² is OH, alkyl C₁-C₆ or aryl C₆-C₁₀;
with the condition that (a) at least one of R¹ to R⁸ is SO₃R⁹, and (b) at least one of the remaining R¹ to R⁸ is an OR¹⁰, CO₂R¹⁰, NR¹⁰R¹¹ or NH-CO-R¹² group, with R⁹, R¹⁰, R¹¹ and R¹² having the previously-mentioned meanings,
or one of the pharmaceutically acceptable salts or prodrugs thereof, in the preparation of a medication for the treatment of vasoproliferative ocular diseases.

2. Use as set forth in claim 1 of a compound of formula (I) according to claim 1 wherein:
A is CR⁸;
R¹ is H or alkyl C₁-C₆;
R² is SO₃R⁹;
R³ is H, OH or alkyl C₁-C₆;
R⁴, R⁵, R⁶, R⁷ and R⁸, independently of each other, represent H, OR¹⁰, CO₂R¹⁰, NR¹⁰R¹¹, NH-CO-R¹² or alkyl C₁-C₆;
R⁹ is H, ammonium or a cation of an alkaline or earth alkaline metal;
R¹⁰ and R¹¹, independently of each other, represent H, alkyl C₁-C₆ or aryl C₆-C₁₀;
R¹² is OH, alkyl C₁-C₆ or aryl C₆-C₁₀;
with the condition that at least one of R⁴, R⁵, R⁶, R⁷ or R⁸ is OR¹⁰, CO₂R¹⁰, NR¹⁰R¹¹ or NH-CO-R¹², with R¹⁰, R¹¹ and R¹² having the previously-mentioned meanings.

3. Use as set forth in claim 2 of a compound of formula (I) wherein:
A is CR⁸, with R⁸ being H or OH;
R¹ is H;
R² is SO₃R⁹, with R⁹ being H or sodium;
R³ is H;
R⁴, R⁵, R⁶ and R⁷, independently of each other, represent H, OH, OCH₃, COOH, NH₂, NHCOCH₃, NHCOC₆H₅, NHCH₃ or N(CH₃)₂;
with the condition that at least one of R⁴, R⁵, R⁶ or R⁷ is OH, OCH₃, COOH, NH₂, NHCOCH₃, NHCOC₆H₅, NHCH₃ or N(CH₃)₂.

4. Use as set forth in claim 1 of a compound of formula (I) according to claim 1 wherein:
A is CR⁸;
R¹ is SO₃R⁹;
R² is H or alkyl C₁-C₆;
R³ is H, OH or alkyl C₁-C₆;
R⁴, R⁵, R⁶, R⁷ and R⁸, independently of each other, represent H, OR¹⁰, CO₂R¹⁰, NR¹⁰R¹¹, NR-CO-R¹² or alkyl C₁-C₆;
R⁹ is H, ammonium or a cation of an alkaline or earth alkaline metal;
R¹⁰ and R¹¹, independently of each other, represent H, alkyl C₁-C₆ or aryl C₆-C₁₀;
R¹² is OH, alkyl C₁-C₆ or aryl C₆-C₁₀;
with the condition that at least one of R⁴, R⁵, R⁶, R⁷ or R⁸ is OR¹⁰, CO₂R¹⁰, NR¹⁰R¹¹ or NR-CO-R¹², with R¹⁰, R¹¹ and R¹² having the previously-mentioned meanings.

5. Use as set forth in claim 4 of a compound of formula (I) wherein:
A is CR⁸;
R¹ is SO₃R⁹, with R⁹ being H or sodium;
R² is H;
R³ is H or OH;
R⁴, R⁵, R⁶, R⁷ and R⁸, independently of each other, represent H, OH, OCH₃, COOH, NH₂, NHCOCH₂, NHCOC₆H₅, NHCH₃ or N(CH₃)₂;
with the condition that at least one of R⁴, R⁵, R⁶, R⁷ or R⁸ is OH, OCH₃, COOH, NH₂, NHCOCH₃, NHCOC₆H₅, NHCH₃ or N(CH₃)₂.

6. Use as set forth in anyone of claims 1, 2, 3, 4 or 5 of a compound selected from the group comprised by sodium 5-amino-2-naphthalene sulfonate, sodium 5-acetyl amino-2-naphthalene sulfonate, sodium 5-benzoyl amino-2-naphthalene sulfonate, sodium 8-amino-2-naphthalene sulfonate, sodium 8-acetyl amino-2-naphthalene sulfonate, sodium 4-hydroxy-6-amino-2-naphthalene sulfonate, sodium 4-amino-1-naphthalene sulfonate, sodium 4-acetyl amino-1-naphthaleene sulfonate, sodium 4-benzoyl-amino-1-naphthalene sulfonate, sodium 5-dimethyl amino-1-naphthalene sulfonate and sodium 4-amino-3-hydroxy-1-naphthalene sulfonate and mixtures thereof.

7. Use as set forth in claim 1 of a compound of formula (I) according to claim 1 wherein:
A is N;
R¹, R², R³, R⁴, R⁵, R⁶, R⁷ and R⁸, independently of each other, represent H, SO₃R⁹, OR¹⁰, CO₂R¹⁰, NR¹⁰R¹¹, NH-CO-R¹² or alkyl C₁-C₆;
R⁹ is H, ammonium or a cation of an alkaline or earth alkaline metal;
R¹⁰ and R¹¹, independently of each other, represent H, alkyl C₁-C₆ or aryl C₆-C₁₀;
R¹² is OH, alkyl C₁-C₆ or aryl C₆-C₁₀;
with the condition that (a) at least one of R¹ to R⁸ is SO₃R⁹, and (b) at least one of the remaining R¹ to R⁸ is an OR¹⁰, CO₂R¹⁰, NR¹⁰R¹¹ or NH-CO-R¹² group, with R⁹, R¹⁰, R¹¹ and R¹² having the previously-mentioned meanings.

8. Use as set forth in claim 1, wherein the vasoproliferative ocular disease is a proliferative diabetic retinopathy.

9. Use as set forth in claim 1, wherein the vasoproliterative ocular disease is a wet macular degeneration.
